# EUROPEAN PATENT APPLICATION

(11) **EP 0 574 027 A2**
(43) Date of publication of application: **15.12.1993**
(21) Application number: 93109614.3
(22) Date of filing: 14.06.1993
(51) Int. Cl.: G01N 30/46, G01N 33/497

(54) **Expired gas analytical method and device**

(30) Priority: 12.06.1992 JP 178994/92
(71) Applicant: Ueda, Hideo, Minoh-shi, Osaka (JP); KYOTO DAIICHI KAGAKU CO., LTD., Kyoto-shi Kyoto-fu (JP)
(72) Inventor: Ueda, Hideo, Minoh-shi, Osaka (JP); Hiromoto, Mitsuo, Uji-shi, Kyoto (JP)
(74) Representative: Dipl.-Ing. H. Hauck, Dipl.-Ing. E. Graalfs, Dipl.-Ing. W. Wehnert, Dr.-Ing. W. Döring, Dr.-Ing. N. Siemons

(57) **Abstract**

To expedite breath gas analysis using gas chromatography, a sample from a patient is passed to two columns (51,52) maintained under different chromatographic conditions eg. low and high temperature. The effluent from both columns is then passed to a detector (53). In this way components such as acetone and 4-heptanone which have very different retention times on a single column, can be determined efficiently in a short time without deterioration of chromatographic resolution. The system is used in clinical analysis.

## Description

### BACKGROUND OF THE INVENTION

Conventionally, expired gas has almost not been used as samples in clinical biochemical examination. This is firstly because there is a prejudice in the art that expired gas could not be usable as samples for the clinical biochemical examination, and secondly because specific gas components contained in the expired gas to be detected are quite low in concentration (ppb or ppm at the most) so that they could be measurable only by a special equipment and an instrument (a large-scaled gas detecting device of high sensitivity and a concentrating device of the trace amounts of gas components) and a skilled operator for the measurement. There are heard only few clinical reports of this kind of measurement. Such clinical reports the present inventor knows are (1) Dubowski, K,M, Breath Analysis as a Technique in Clinical Chemistry, Clin .Chem., 20.966-972, 1974, (2) Manolis,A., The Diagnostic Potential of Breath Analysis, Clin.Chem., 29. 5-15, 1983, and (3) Phillips, M., Breath Tests in Medicine, Scientific American July.1992. These reports disclose measurement and observation data only.

Expired gas is intermittently breathed out by human (or animals) during their lives are maintained, and it is readily collectable, without causing subjects a physical or mental pain, particularly from infants, patients with serious illness or damages in consciousness by use of a specially designed instrument. Also, since trace amounts of volatile components of mixed venous blood flowing through alveolar blood capillary is moved into expired gas by gas exchange, it is inferred that expired gas and blood have correlation with respect to the volatile components.

In the meantime, samples used now in most of the clinical examinations are blood. But, collecting blood causes subjects or patients a physical pain with loss of their important blood, and repeated collection of blood and a continuous measurement thereof impose a heavy burden on the subjects or patients. In addition, analyzing the blood samples is delt exclusively or centrally by a blood analysis center (firms specialized in analyzing blood) to occupy much time from collecting blood to the analysis, leading to larger errors in measurement of certain components of blood which easily gasified or denatured. Hence, such unstable components are exceptionally measured only at large-scaled hospitals provided with special analyzing apparatuses. Meanwhile, urine is discharge relatively readily and amply collectable and broadly used in screening, but has a problem that it provides less information than blood does. Also, the examinations with urine cause the subjects or patients a mental pain as from a feeling of shame and do not enable a continuous measurement, and urine is hard to be collected at all times.

Under the circumstances, the inventors have zealously studied development of clinical examination methods and devices using expired gas as samples. Expired gas when collected in vessels occupies much spaces for preservation and transportation, so that they are not well subjected to the same analyzing course as that of blood, i.e., as first transported to the analysis center and analyzed by the large-scaled apparatuses. Also, in consideration of convenience in use for a bedside examination, a pre-hospital examination in an ambulance, screening upon medical care and the like, the examination device is required to be small-sized, portable, of high sensitivity, simply operable and superior in safety and rapidity in measurement. Reliability of provided data and economization in use of such examination device are also required.

The inventors have previously developed a practical clinical examination method and device using expired gas as samples under the foregoing circumstances. The previously developed examination method and device causes gas components contained in expired gas to be separated through a column and detects the components by a detector. The detector employs PID (Photo Ionization Detector), IMS (Ion Mobility Spectrometer) or ECD (Electron Capture Detector), wherein the aimed gas components to be detected is applied with light or radiation to be ionized so that measurement signals are output corresponding to amounts of ionization of the gas components. This type of detector does not have burning of hydrogen gas as in FID (Flame Ionization Detector) or FPD (Flame Photometric Detector) to thereby be safe and small-sized, and also of high sensitivity and high accuracy in comparison with FID and FPD, and further having an advantage that air or nitrogen gas which is cheap can be used as carrier gas.

Also in examinations with expired gas similarly with the cases with blood or urine, measurement of a plurality of gas components may be required occasionally, for example, such cases as screening for a plurality of disease items, or the case that a specific gas component contained in expired gas which gas component relates to many kinds of diseases is measured together with other gas components for diagnosing the diseases, also or the case that a plurality of gas components having a clinical significance are measured. Since the specific gas components are occasionally largely different to each other in their retention time, the previously developed examination device when used to measure these gas components at a time will take a longer time for each measurement operation, thereby not sufficing for urgent measurement or screening. In detail, in case of the gas components, for example, acetone and 4-heptanone detailed later, retention time of acetone is 50 sec under a certain separation condition, and that of 4-heptanone 1860 sec (31 min). This leads to such redundant and inefficient use of the examination device that when a number of samples are to be measured, it takes many hours.

### SUMMARY OF THE INVENTION

An object of the invention is to provide a novel expired gas analytical method and device for clinical purpose by which analytical method and device a plurality of gas components contained in expired gas of subjects can be measured rapidly and accurately with a single operation by use of gas chromatograph. Another object of the present invention is to provide an expired gas analytical device which is of high sensitivity, readily operable, applicable to infants, aged men and women and unconscious patients and also having safety, reliability of provided data and economization in use of the device.

### BRIEF DESCRIPTION OF THE DRAWINGS

Fig. 1 is a block diagram showing an example of an expired gas analytical device according to the present invention.

Fig. 2(a) is a gas chromatogram obtained by separating an expired gas sample with a column for separating acetone, and Fig. 2(b) a gas chromatogram obtained by separating an expired gas with a column for separating 4-heptanone.

Fig. 3 is a gas chromatogram of an expired gas measured by the expired gas analytical device according to the present invention.

Fig. 4 is a block diagram showing a modified example of the expired gas analytical device of the invention.

### DETAILED DESCRIPTION OF THE INVENTION

To overcome the aforesaid problems, the inventors have provided in the expired gas analytical device a plurality of columns different in gas separating conditions (shapes of columns, adsorbents and column temperatures) so that a plurality of gas components, which may have large difference from each other in retention time under the same separating condition, can be measured in a short time with less difference in the retention time without deterioration of separation accuracy.

The inventors' disposal of the problems will be explained with referring to measurement of ketone body contained in expired gas. Conventionally, measurement of ketone body means that of acetoacetic acid contained in blood and urine. That is, acetoacetic acid (AcAc) increases due to sthenia of fat metabolism in diabetes (type I) or bulimia (acetonemic vomiting, etc) and is made use of for diagnosing the diseases. 3-hydroxybutyric acid (3-OHBA) is also measured rarely and acetone (Ac) formed by decarboxylation of acetoacetic acid is hardly measured since acetone is discharged into expired gas to be in quite trace amount.

In addition to the above, there made such reports in recent years that a novel fat metabolite (ketone body), i.e., 4-heptanone is possible to exist in urine. (Liebich H.M. Gas chromatographic-Mass Spectrometric Determination of Total 4-heptanone, A New Marker in Diabetes Mellitus: Journal of Chromatography, vol 273, 67-75, 1983. Liebich H.M. Gas chromatographic Profiling of Ketone Bodies and Organic Acids in Diabetes, ibid, vol 379, 347-366, 1986.). According to the report, the three components hitherto called ketone body (i.e., acetone, acetoacetic acid and 3-hydroxybutyric acid) increase similarly to each other in ketoacidosis or the like, and 4-heptanone serving as the new marker increases when ketoacidosis takes a turn for the better and those three components reduce. In detail, (1) In glycophilia and ketoacidosis, the three components are high in concentration and 4-heptanone is low in concentration, (2) In the state (1), when the condition of disease is well controlled by alimentary therapy or the like, the three components are low in concentration and 4-heptanone high, and (3) In case of healthy subjects, the thee components and 4-heptanone are low in concentration.

The inventors carried out measurement of expired gas on the basis of the knowledge and obtained the same measurement results as the above. To be noted is that since acetone among the conventional three components in ketone body shows higher concentration in measurement of expired gas, we measured acetone as a representative component of the three components. Measurement of ketone body in blood or urine takes much cost and labor while the analysis of ketone body by using expired gas is quite simple. Hence, measuring acetone and 4-heptanone in expired gas has a large significance for diagnosis of diabetes and as a monitor for therapy.

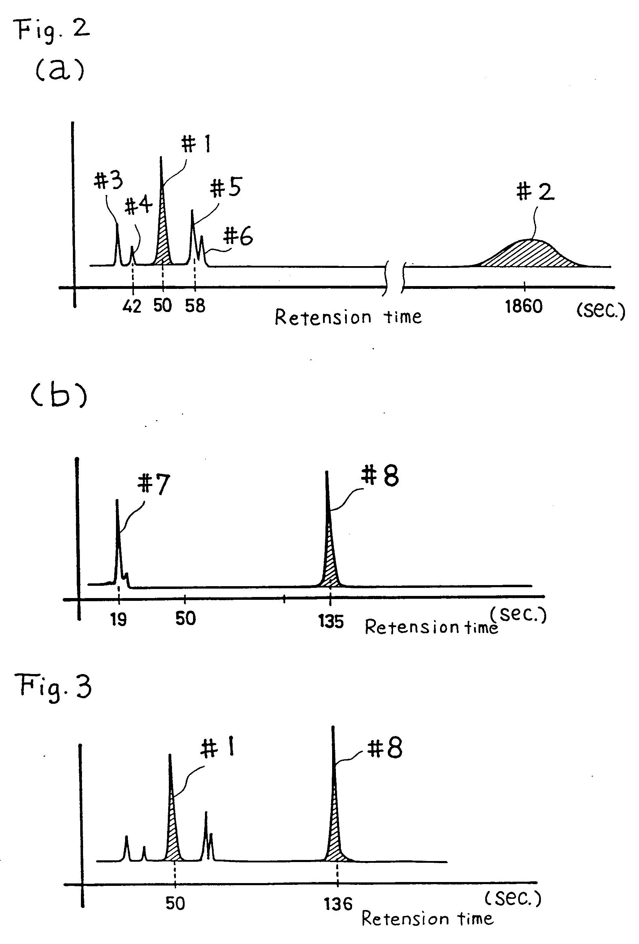

Fig. 2(a) shows the gas chromatogram obtained by measuring acetone and 4-heptanone in expired gas with using an expired gas analytical device (a single column: column temperature of 60°C) previously developed by the inventors. As seen in the drawing, retention time of acetone (peak #1) is 50 sec and that of 4-heptanone (peak #2) 1860 sec (31 min). The difference of retention time is inferred to be from difference of boiling points, i.e., 56.5°C for acetone and 150°C for 4-heptanone and causes the analytical device to have no practicality as a clinical biochemical examination device. In Fig. 2(a), peak #3 is acetaldehyde, peak #4 isoprene, peak #5 ethanol, and peak #6 isopropanol. Fig. 2(b) is a gas chromatogram similarly obtained by the expired gas analytical device with temperature of the single column being set to 150°C, wherein retention time of 4-heptanone (peak #8) is substantially reduced to 135 sec (2.25 min) while acetone is measured but not clearly separated from other low boiling point compounds, such as acetaldehyde and others, (peak #7), thereby making impossible an accurate measurement.

The present invention has been designed to solve this problem and has a characteristic in that the expired gas analytical device is constructed with a multi-channel style using a plurality of columns differing in gas-separating conditions. For the above case of ketone body, we used two columns each set to 60 and 150°C respectively without changing adsorbents and column shapes. Generally, a low temperature column may be selectively set to 40 to 60°C and a high temperature column 100 to 200°C depending upon specific gas components to be measured. Optimum column shapes and adsorbents in addition to column temperatures may be selected for aimed gases to further reduce the retention times. Also, three or more columns may be usable in combination with selected column temperatures and structures and fillers.

A plurality of columns may be preferably used for examination of various diseases in addition to diabetes, for example, liver cirrhosis and also for examination of newborn infants. Expired gas of a patient with serious liver cirrhosis contains low-boiling ammonia (-33.4°C) and methyl mercaptan (6°C) and high-boiling lower fatty acid such as acetic acid (118.1°C), propionic acid (141.1°), isobutyric acid (154.5°C), butyric acid (163.5°C) and isovaleric acid (176°C). To rapidly and accurately separate and measure those gases, a low temperature column (e.g., 40°C) and a high temperature column (e.g., 150°C) are required. Also, when an inborn error of metabolism of newborn infants, such as hyperammonemia, phenylketonuria, or isovalericacidemia, is examined by inspecting existence of the gases in expired gas of the newborn infants, a low temperature column (for ammonia) and a high temperature column (for the remainder) may be used to carry out measurement accurately and rapidly.

Next, the expired gas analytical device according to the present invention developed for use in the foregoing expired gas examinations will be detailed. The expired gas analytical device generally comprises an expired gas breathe-into part, a carrier gas feeding part, a sample measuring part, a detecting part and an arithmetic processing part. The detecting part (i.e., a column and a detector) does specially employ a particular construction so as to enable rapid measurement with high sensitivity and high accuracy and be small-sized as required. The column may use a packed column or a capillary column. The capillary column is more preferably used since it requires less amount of samples and provides a sharp chromatogram. A plurality of columns are mounted, each column being set of constructions, kinds of adsorbents (fillers and liquid layers) and heating temperatures corresponding to aimed gas components so as to allow retention time of the aimed gas components to be several minutes or 2 to 3 min. The adsorbents are selected, corresponding to specific gases to be detected, from various kinds of fillers and liquid layers hitherto known in consideration of such conditions required for the examination as measurement accuracy, reproducibility and rapidity.

The detector employs the foregoing PID, IMS or ECD. The photo ionization detector (PID) which does not use radiation is most preferable. The PID utilizes the phenomenon that an aimed gas component is applied with light (ultraviolet) having energy larger than ionization potential of the gas, thereby causing ionization. An amount of ionization of the gas is converted to ionizing current with electrodes to be output, so that concentration of detected gas components is determined from magnitude of ionizing current. The detector may be used in the same number as the columns, but a single detector is sufficient when aimed gas components have large difference in retention time.

The expired gas breathe-into part is adapted to feed expired air (expired gas) to the sample measuring part and comprises an expired gas collector such as a mouthpiece or a mask for collecting expired gas and a collecting tube (a sampling probe) mounting the expired gas collector at one end. The expired gas breathe-into part, particularly, the collecting tube is preferably to be heated at its inner surface to human body temperature or higher temperatures, for example, 36 to 100°C, more preferably 40 to 50°C. This prevents that moisture of the expired gas condenses on and sticks to an inner wall of the collecting tube to dissolve and adsorb an aimed gas component. For heating the collecting tube, a heating element may be disposed circumferentially of or inside the collecting tube, or the tube may be made of a material having itself heat build-up and sheathed with insulating material. Also, the collecting tube may include a temperature adjuster mechanism. The expired gas collector may be disposable type to be sanitary.

Such feature may be adopted that expired gas is collected first in a separate expired-gas collecting means such as a large injector or a gas bag (but not directly breathed into the analytical device) and then fed into the device through the collecting tube. Alternatively, expired gas collected at a predetermined amount by a syringe may be injected from an expired gas sample injecting part formed at the upper stream side of the column. In these cases, the expired gas collecting means, i.e., the injector, gas bag and syringe requires to be kept at specific temperatures or heated so that inside of the expired gas collecting means can be kept at temperatures higher than human body temperature.

The sample measuring part collects and holds as an expired gas sample a predetermined amount of portion of expired gas fed from the expired gas breathe-into part. The collection of the predetermined amount of air portion may be carried out by patient's positive blowing to push the expired gas or preferably by use of a sampling pump to take in or press the expired gas. Use of the sampling pump has advantages that expired gas can be collected readily from infants or unconscious patients and contamination (carry-over) on the inner wall of the collecting tube due to a previous subject's expired gas can be purged and removed by taking in atmosphere or a subsequent patient's expired gas.

The sample measuring part may be so constituted that a carrier gas passage is provided partially with two sets of valves to form therebetween a measuring chamber whose front end connects with an end of the collecting tube through a valve. Also, the sample measuring part may use a known six-way valve (injection valve) or be so constructed that an end of the collecting tube is connected to a cylinder serving as the measuring chamber to feed into the carrier gas passage an expired gas sample collected in the measuring chamber.

Since the invention uses a plurality of columns, the sample measuring parts may be provided in the same number as the columns for highly accurate measurement. Alternatively, a single set of sample measuring part may be provided to allow expired gas sample to be fed equally to the columns, or a change-over valve may be provided between the sample measuring part and the column group to feed the expired gas sample to the columns in order. A predetermined amount of expired gas sample to be fed into each column depends upon capacity of the detecting part and may be about 0.05 to 5.0 ml, more preferably 0.1 to 0.8 ml. The sample measuring part, particularly, the measuring chamber needs to be kept at a constant temperature higher than human body temperature (for example, the same temperatures as the collecting tube) to prevent sticking of moisture and keep constant the mass of the expired gas samples.

The carrier gas feeding part feeds a carrier gas which sends the expired air samples to the separation column. A source of the carrier gas is preferably a small-sized gas cylinder in consideration of the portability of the analytical device according to the invention. In case that the analytical device is installed at a fixed position for use, a larger gas cylinder is usable for the carrier gas source. Also, the invention can employ as the carrier gas a purified air or nitrogen gas which are cheap, and also all kinds of gases, such as helium or the like, generally used in the art. In case of using air as the carrier gas, ambient air not the air packed in a cylinder may be used to be fed by a compression pump. In this case, an air filter including adsorbent or the like is provided for purifying taken ambient air to eliminate effects of trace amounts of gas content of the taken ambient air.

A principal portion of the arithmetic processing part is a microcomputer which controls an operating program for the whole of the expired air analytical device as receives measurement signals output from the detector to process the signals, computes concentration of an aimed gas component by use of previously memorized calibration curves, stores the computed concentration as a clinical examination data or outputs the data on an indicating device (display) or a recording device (printer), or receives input signals from a keyboard.

Use of the analytical device constructed as above according to the present invention is as follows.
(1) The sample collector is first put to or on patient's mouth or face. The measurement start button on the keyboard is depressed and simultaneously the patient is caused to blow breath for few seconds. (When a subject (patient) stops breathing about 10 to 20 sec before blowing breath through the collector and a very small amount of expired gas just blown is discarded, remainder can be regarded as alveolar air component equilibrating with mixed venous blood gas partial pressure, thereby enabling measurement with excellent reproducibility.
(2) The expired gas is sucked through the collecting tube and a part of the expired gas is collected in the measuring chamber and fed into the respective columns by carrier gas.
(3) Trace amounts of aimed gas components of the expired gas are developed and separated in the columns, and the gas components having shorter retention time are first ionized in the detector and an amount of ionization of the gas is detected with high sensitivity.
(4) Output from the detector is arithmetically processed and concentration of the aimed gas components are computed on the basis of previously memorized calibration curves and stored as a clinical examination data or output on the outputting device. Each measurement ends in several minutes.

The analytical device of the invention, particularly, those provided with a sampling pump is adapted to automatically make operation by itself after subjects merely blow their breath in the device. Hence, the examination can be readily applied to any subjects such as aged men and women, newborn infants, and unconscious patients. Furthermore, since the analytical device is small-sized and readily operable and enables measurement in a short time without causing patients any pain, the device is quite suitable for not only a sudden examination of diabetes but also a continuous examination such as tolerance test or monitoring of a course of diseases.

### EMBODIMENTS

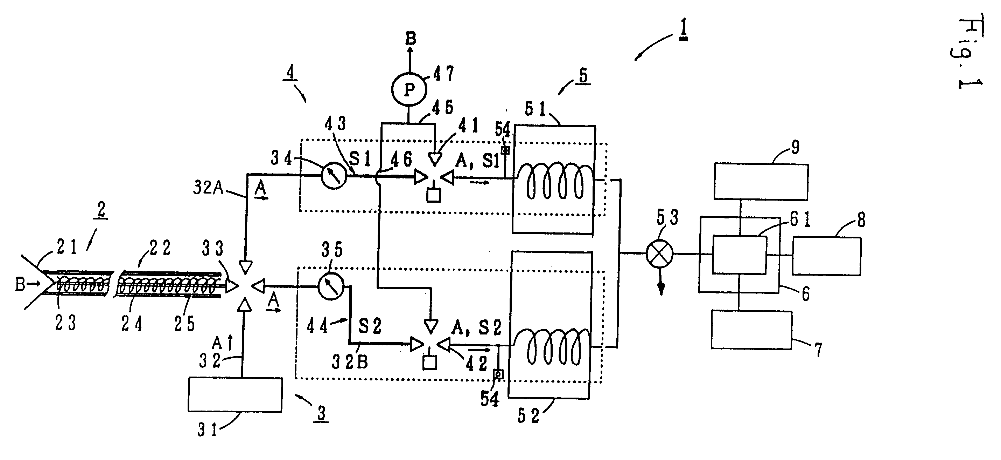

Next, the invention will be detailed with referring to the examples. To be note is that the present invention should not at all be limited to the examples. Fig. 1 is a block diagram showing an expired gas analytical device 1 according to the present invention. The analytical device 1 comprises an expired gas breathe-into part 2, a carrier gas feeding part 3, a sample measuring part 4, a detecting part 5, an arithmetic processing part 6, a keyboard 7 serving as an input device, a printer 8 serving as an output device and a display 9. The detecting part 5 is provided with two columns each separating acetone and 4-heptanone, respectively and two sample measuring chambers.

The expired gas breathe-into part 2 comprises an expired gas collecting mask 21 serving as an expired gas collector and a collecting tube 22 mounting the mask 21 at the utmost end. The collecting tube 22 comprises a Teflon pipe 23 (1 - 5 mm in inner diameter and about 1.5 m in length) mounting thereon a heater 24 and sheathed with a heat insulating material 25, so that the pipe 23 is heated of the inside to prevent moisture of expired air from sticking on the inner surface of the pipe 23. The heating is carried out at a temperature of 36 to 100°C, for example, 40°C adjusted by a controller. A mouthpiece may be usable instead of the expired gas collecting mask 21. The collecting tube 22 is connected at its rear end to a four-way solenoid valve 33.

The carrier gas feeding part 3 comprises a small-sized air cylinder 31 containing purified air, a carrier gas passage 32 and the four-way solenoid valve 33. The carrier gas passage 32 made of Teflon pipe or the like connects the air cylinder 31 with the four-way solenoid valve 33 and diverges at the solenoid valve 33 into two lines leading to the respective columns. The carrier gas passage branch 32A and 32B may be provided with a flow adjuster 34, 35.

The sample measuring part 4 is defined by a part of the carrier gas passage 32. In detail, each of the carrier gas passage branches 32A, 32B from the four-way solenoid valve 33 are locally provided with a three-way solenoid valve 41, 42, respectively to form a measuring chamber 43 for acetone by the part defined by the solenoid valves 33 and 41 and a measuring chamber 44 for 4-heptanone by that defined by the solenoid valves 33 and 42. The solenoid valves 41 and 42 are connected with a suction pump 47 through a discharge tube 45, 46. The carrier gas passage branches 32A, 32B are constructed similarly with the collecting tube 22 by use of the same materials, so that the carrier gas passage branches 32A, 32B are similarly heated at about 40°C. A capacity of the measuring chambers 43, 44 is about 0.5 ml but may be set separately corresponding to specific gas-separation conditions. In case that the suction pump 47 is omitted, a patient (subject) may blow through the collecting tube to force his or her expired gas inside.

In case that a predetermined amount of expired gas is collected in a syringe in place of the subjects' direct blowing breath in the collecting tube (including the case using the gas bag or the large-sized injector), such a modified feature may be used that instead of the expired gas breathe-into part 2, an expired gas sample injecting part 54 is formed at the upper stream side of the columns, through which part 54 a measured expired gas sample is fed into the columns.

The detector 5 comprises a column 51 for separating acetone, a column 52 for separating 4-heptanone and a single detector 53. The acetone-separation column 51 may employ a capillary column (PEG-20M, 0.25 mm⌀ x 25m, film thickness of liquid in solid phase: 0.25µm) and carries out gas-separation at column temperature 60°C with carrier gas flow of 2 ml/min. Another column 52 for 4-heptanone employs the same column as that for acetone and carries out gas-separation at a set temperature 150°C. The separated gas components fed from the columns join together and are fed into the single detector 53 which is a photo ionization detector (PID). Two detectors may be used (not shown). 4-heptanone or other gases which have longer retention time, separated in the acetone-separation column may be removed by backflush.

The microcomputer 61 of the arithmetic processing part 6 when receives measurement signals from the detector processes the signals, computes concentration of acetone and 4-heptanone by use of previously memorized calibration curves, outputs the computed concentration as a clinical examination data on the printer 8 the display 9. Fig. 2(a) is a gas chromatogram of an expired gas sample provided only with the acetone-separation column 51. As seen from the drawing, the peak of acetone (peak #1) clearly appears at retention time about 50 sec as accurately separated from the other gas components of the expired gas such as acetaldehyde, isoprene, ethanol and isopropanol having approximate properties to acetone. Fig. 2(b) is a gas chromatogram of an expired gas sample analyzed by using only the column 52 for 4-heptanone, wherein 4-heptanone (peak #8) clearly appears at retention time about 2 min while acetone and the other low-boiling point compounds do not show sufficient separation (peak #7).

The analytical device in the example uses the two columns in combination and the single detector 53 but is set to allow acetone and 4-heptanone to have difference in retention time, so that acetone and 4-heptanone are detected in order by the detector 53 without overlapping with each other and the gas chromatogram as shown in Fig. 3 is obtained. The gas chromatogram in Fig. 3 corresponds to a synthesis of the gas chromatograms (charts) of Figs. 2(a) and 2(b). To be noted is that the clinical examination data obtained by the analytical device may be stored in the microcomputer 61 or output on the printer or display as aforesaid.

In use of the analytical device, a main switch on the keyboard 7 is first turned on to have a constant temperature at the specific parts. The expired gas collecting mask 21 is fit on the patient's face and the measurement start button on the keyboard 7 is depressed. Expired gas B is exhausted outside by the suction pump 47 while a part of the expired gas B is filled in the measuring chambers 43, 44 to be collected as a predetermined amount of expired gas sample S1 (for analyzing acetone) and that S2 (for 4-heptanone) by closing the solenoid valves 33, 41 and 42. A subject's blowing expired gas is about 5 sec. Each expired gas sample S1 and S2 is fed into the respective column 51 and 52 by the carrier gas A (sent from the air cylinder 41 or the like) to be developed, separated and fractionated depending on specific retention time of the gases, and ionized orderly in the detector 53. Amounts of ionization of acetone and 4-heptanone are converted to electric signals to be output. The electric signals are processed by the arithmetic processing part 6 so that concentration of acetone and 4-heptanone are determined by use of previously memorized calibration curves and memorized and displayed. Measurement ends about 3 min after the expired gas of the subject is sucked into the device.

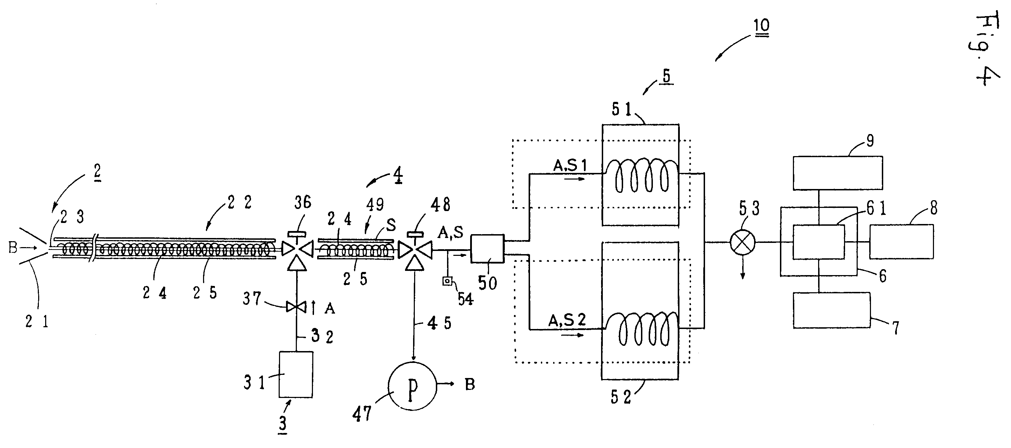

The expired gas analytical device 10 shown in Fig. 4 comprises a single measuring chamber 49, columns 51 and 52 in parallel to each other and a distributor or a change-over means 50 interposed between the measuring chamber 49 and the columns. The measuring chamber 49 comprises an extension of the collecting tube 22 partitioned or sandwiched by two sets of three-way solenoid valves 36 and 48. Structures other than the above are the same as those of the analytical device in the foregoing example shown in Fig. 1.

### EFFECTS OF THE INVENTION

As clearly seen from the above, the present invention relates to an expired gas analytical device which analyzes an expired gas sample of subjects to measure aimed trace amounts of gases contained in the expired gas sample. An expired gas sample fed from the expired gas breathe-into part is sent through the columns to the detector which ionizes trace amounts of aimed gas components contained in the expired gas by applying ultraviolet or radiation to the aimed gas components so as to detect such gas components, thereby measuring concentration of the gas components. Hence, the invention has the following characteristics.
(1) In an analysis of expired gas, a plurality of gas components in the expired gas which gas components have large difference in retention time under the same gas-separation conditions can be measured rapidly with high accuracy and reproducibility without being influenced by other components. Hence, the analytical device can be further effectively applicable to examination, for example, of ketone body for which the conventional analytical method with urine has a problem of sensitivity in detection and that with blood takes much cost and labor.
(2) The invention uses as sample the expired gas which is bloodless and noninvasive and does not cause a patient a pain, a feeling of fear or pressure. Hence, a burden imposed on the patient in relation to repeated measurement for tolerance test and a continuous observation can be completely eliminated. Also, since the analytical device provides examination results instantly, diseases can be located at an early stage.
(3) Since a detector which needs no burning of hydrogen and enables measurement with high sensitivity in a short time is used, the analytical device can be made small-sized, readily operable and enable rapidity of measurement with a quite low cost for the examination.
(4) Operation of the device is merely causing the subjects to blow their breath through the expired air collector. Hence, an operator does not need to have a special training for use the device. Also, the device provided with a sampling pump can readily collect an expired gas sample from infants or patients with serious illness or unconsciousness.

## Claims

1. An expired gas analytical device serving as a clinical examination device for fractionally measuring by use of gas chromatography gas components contained in the expired gas fed from an expired gas breathe-into part or an expired gas sample injecting part, the expired gas analytical device including a plurality of columns whose gas-separation conditions such as adsorbents for the columns and column temperatures are so set corresponding to aimed gas components that a plurality of gas components which have a large difference in retention time under the same gas-separation condition are provided with specific retention times approximated to each other and to a shorter retention time without deterioration of gas-separation accuracy.

2. An expired gas analytical device as set forth in claim 1, wherein a sample measuring part for measuring expired gas samples is provided in the same number as the columns.

3. An expired gas analytical device as set forth in claim 1, wherein a set of a sample measuring part for measuring expired gas samples is provided, and a distributor or a change-over means is interposed between the sample measuring part and a plurality of columns in parallel to each other.

4. An expired gas analytical device as set forth in claim 1, 2 or 3, wherein a plurality of columns are provided having higher and lower selective temperatures.

5. An expired gas analytical method fractionally measuring a plurality of gas components contained in expired gas samples by use of gas chromatography, wherein expired gas samples fed from an expired gas breathe-into part or an expired gas sample injecting part are introduced into a plurality of columns having different gas-separation conditions, so that a plurality of gas components which have large difference in retention time under the same gas-separation condition are measured in a short time with reduced difference in retention time without deterioration of gas-separation accuracy.
